## Europäisches Patentamt
## European Patent Office
## Office européen des brevets

(11) Veröffentlichungsnummer: **0 041 693**
**B1**

(12) # EUROPÄISCHE PATENTSCHRIFT

(45) Veröffentlichungstag der Patentschrift:
**16.10.85**

(51) Int. Cl.⁴: **A 61 B 5/10**

(21) Anmeldenummer: **81104280.3**

(22) Anmeldetag: **03.06.81**

(54) **Kapazitiver Sensor zur Erfassung von Fingerabdrücken.**

(30) Priorität: **05.06.80 US 156571**

(43) Veröffentlichungstag der Anmeldung:
**16.12.81 Patentblatt 81/50**

(45) Bekanntmachung des Hinweises auf die Patenterteilung:
**16.10.85 Patentblatt 85/42**

(84) Benannte Vertragsstaaten:
**BE DE FR GB IT NL**

(56) Entgegenhaltungen:
**DE - A - 2 529 475**
**FR - A - 2 340 547**
**US - A - 3 622 989**
**US - A - 3 781 855**
**US - A - 3 973 146**

(73) Patentinhaber: **Siemens Aktiengesellschaft, Berlin und München Wittelsbacherplatz 2, D-8000 München 2 (DE)**

(72) Erfinder: **Tsikos, Constantine, 5316 King Avenue, Pennsauken, NJ 08109 (US)**

**Beschreibung**

Die vorliegende Erfindung betrifft einen Sensor zur Erfassung von Fingerabdrücken mit einer Abtastoberfläche, gegen die der abzutastende Finger gepresst wird, mit Mitteln zum Abtasten der Erhebungen und Vertiefungen in der Hautoberfläche des Fingers, die eine Vielzahl von Kondensatoren enthalten, die in einer Abtasteinheit zusammengefasst und in Form einer zweidimensionalen Matrix angeordnet sind, wobei jeder Kondensator zumindest eine Elektrode aufweist, die mit einem Eingang eines Multiplexers verbunden ist, und mit einer Speichereinrichtung zum Speichern von Ausgangssignalen der Abtasteinheit.

Bei einem Fingerabdruck-Eingangssignalwandler oder -Sensor wird der abzutastende Finger üblicherweise gegen eine ebene Fläche, beispielsweise eine Seite einer Glasplatte, gepresst, wobei der abzutastende Finger durch Abtastmittel, beispielsweise durch einen abtastenden Lichtstrahl, abgetastet wird. Die Auswertung der Fingerabdruck-Information kann beispielsweise mittels Laser-Technik vorgenommen werden.

Aus der DE-A-2 529 475 ist ein Sensor für die Erfassung von Kräfteverteilungen, die der menschliche Körper auf eine Unterlage ausübt, bekannt, bei dem eine Vielzahl von druckempfindlichen Kondensatoren vorgesehen sind, welche zur Erzielung einer hohen Flächenauflösung sehr klein ausgebildet sind und deren Kapazitäten örtlich in Übereinstimmung mit dem ausgeübten Druck verändert werden, wenn die Oberfläche des menschlichen Körpers dagegen drückt. Dieser bekannte Sensor stellt eine Matrix aus variablen Scheinwiderständen dar, durch die eine Vielzahl von Wechselströmen fliesst, deren jeweilige Stärke durch die verschiedenen Druckkräfte bestimmt ist. Die erzielbare Messgenauigkeit ist dabei wegen der unvermeidlichen Verkoppelung der Strompfade begrenzt.

Fingerabdruck-Identifikationseinrichtungen der eingangs genannten Art werden im allgemeinen benutzt, um den Zugriff durch Personen zu bestimmten Informationsquellen, beispielsweise über Computer-Endgeräte (Informationszugangskontrolle), oder den Zugang zu Gebäuden (körperliche Zungangskontrolle) zu kontrollieren.

Eine Schwierigkeit im Zusammenhang mit Sensoren zur Erfassung von Fingerabdrücken besteht hinsichtlich der Zuverlässigkeit von der genauen Umsetzung des Musters von Erhebungen und Vertiefungen der Fingerkuppe in elektrische Signale. Optische Techniken, die weitgehend zur Anwendung gelangen, benötigen einen grossen Aufwand von hochwertigen Einrichtungen. Einfache elektromechanische Sensoren sind für einige Anwendungsfälle nicht genügend empfindlich.

Es besteht daher ein Bedarf für einen Fingerabdruck-Eingangssignalsensor oder -wandler, der derart ausgelegt ist, dass er zuverlässig die Reliefs von Fingerkuppen abtasten kann und die jeweils abgetastete Information in elektrische Signale umsetzt.

Der vorliegenden Erfindung liegt die Aufgabe zugrunde, einen Sensor mit den genannten Eigenschaften zu schaffen, der einen einfachen Aufbau besitzt.

Der erfindungsgemässe Sensor soll in der Lage sein, die Information, die in der Hautstruktur eines menschlichen Fingers, insbesondere in der Hautstruktur der Fingerkuppe, enthalten ist, zu erkennen und abzutasten sowie ein elektrisches Ausgangssignal in Übereinstimmung mit dem Muster von Erhebungen und Vertiefungen der Fingerkuppenhaut zu liefern. Der Sensor soll ausserdem schnell arbeiten und nach einer vorangegangenen Fingerabdruck-Überprüfung schnell wiederverwendbar sein. Darüber hinaus soll der Sensor die Fingerabdruck-Information über eine bestimmte Zeitpsanne hinweg speichern können, nach der, falls dies gewünscht wird, die Information ausgelesen oder sichtbar gemacht werden können soll. Ausserdem soll der Sensor weder optische noch elektromechanische Elemente enthalten.

Die der Erfindung zugrundeliegende Aufgabe wird durch einen Sensor für Fingerabdrücke gemäss dem Oberbegriff des Patentanspruchs 1 gelöst, der durch die in dessen kennzeichnenden Teil angegebenen Merkmale charakterisiert ist.

Vorteilhafte Weiterbildungen der Erfindung sind durch die in den Unteransprüchen angegebenen Merkmale gekennzeichnet.

Entsprechend der vorliegenden Erfindung enthält der Sensor für Fingerabdrücke einen Abtastteil, der eine Abtastoberfläche zur Aufnahme des Fingerabdrucks aufweist. Die Abtastoberfläche ist Teil einer Abtasteinheit, die eine grosse Anzahl von kleinen Kondensatoren enthält. Diese Kondensatoren sind in ihren Abmessungen kleiner als die Breiten der Erhebungen und Vertiefungen der Fingerkuppenhaut, um eine hohe Auflösung zu erreichen. Die Kondensatoren sind nahe der Abtastoberfläche in einer zweidimensionalen Anordnung vorgesehen. Wenn der Finger gegen die Abtastoberfläche gepresst wird, ändern sich die Kapazitäten der Kondensatoren örtlich entsprechend dem Erhebungs/Vertiefungs-Muster der Fingerkuppenhaut. Die Änderung der Kapazität kann durch elektrische Mittel erkannt werden. Daraus ergibt sich ein «kapazitiver Sensor zur Erfassung von Fingerabdrücken». Dieser Sensor kann beispielsweise für Zwecke einer persönlichen Zugangskontrolle verwendet werden.

Die genannte Aufgabe und weitere Aufgaben sowie Vorteile der Erfindung ergeben sich aus der im folgenden gegebenen, ins einzelne gehenden Beschreibung im Zusammenhang mit den beigefügten Figuren.

Die im folgenden beschriebenen Figuren zeigen lediglich bevorzugte Ausführungsbeispiele für die vorliegende Erfindung.

Fig. 1 zeigt ein Schaltbild einer Matrix von Kondensatoren in einer Abtasteinheit, wobei einige Gruppen von Kondensatoren in Reihe geschaltet sind.

Fig. 2 zeigt einen vergrösserten Querschnitt durch einen menschlichen Finger, wobei die Erhebungen und Vertiefungen verdeutlicht sind; der Finger wird gegen eine Abtastoberfläche gepresst und erstreckt sich dabei längs einer Achse x.

Fig. 3 zeigt eine eindimensionale Anordnung von Kondensatoren, die in Reihe mit einer Spannungsquelle zum Abtasten des Musters der Fingerkuppenoberfläche gemäss Fig. 2 geschaltet sind.

Fig. 4 zeigt die Verteilung der Kapazitäten der in Fig. 3 dargestellten Kondensatoren als Ergebnis des Andrückens des Fingers gemäss Fig. 2.

Fig. 5 zeigt eine Verteilung der individuellen Spannungen der Kondensatoren, die in Fig. 3 dargestellt sind, als Ergebnis des Andrückens des Fingers gemäss Fig. 2.

Fig. 6 zeigt ein Blockschaltbild einer elektrischen Schaltungsanordnung zum Auslesen und Speichern der individuellen Spannungen der Kondensatoren in der Anordnung gemäss Fig. 1.

Fig. 7 zeigt ein Ausführungsbeispiel für einen Sensor zur Erfassung von Fingerabdrücken, der eine grosse Anzahl von gekrümmten Elektroden aufweist, wobei jeweils zwei einander benachbarte Elektroden einen Kondensator bilden.

Fig. 8 zeigt ein weiteres Ausführungsbeispiel für einen Sensor zur Erfassung von Fingerabdrücken, der eine grosse Anzahl von ebenen Elektroden und eine flexible Elektrode, die den ebenen Elektroden gegenüberliegt, aufweist.

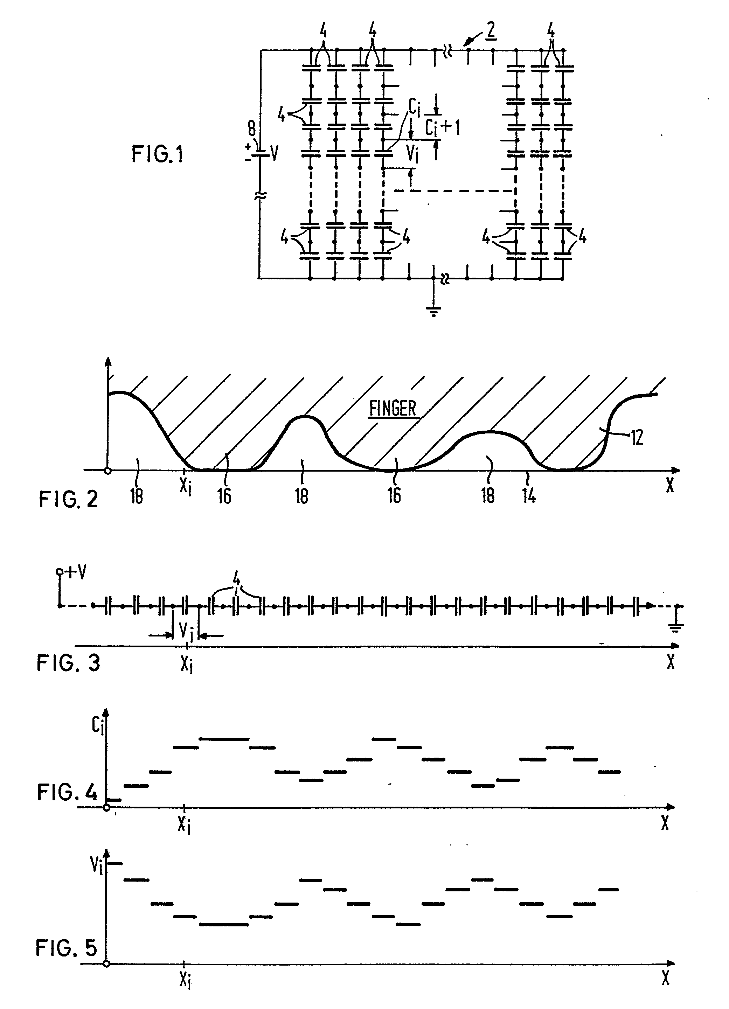

Wie Fig. zeigt, enthält die Abtasteinheit eines Sensors 2 zur Erfassung von Fingerabdrücken eine zweidimensionale Anordnung von Kondensatoren 4. Die Anordnung ist vorzugsweise rechteckig gestaltet. Es können jedoch auch andere Anordnungen gewählt werden. Fig. 1 repräsentiert einen vergrössert dargestellten Ausschnitt der Abtasteinheit. Dem ist zu entnehmen, dass eine grosse Anzahl von Kondensatoren 4 vorhanden ist. Die Kondensatoren sind in ihren Abmessungen kleiner als die Erhebungen und die Vertiefungen in der Fingerkuppe (in Fig. 1 nicht gezeigt), die abgetastet werden soll. Die Grösse der Kondensatoren ist um wenigstens eine Grössenordnung kleiner als die durchschnittliche Breite der Erhebungen und Vertiefungen der Haut an der Fingerkuppe. Alle Kondensatoren 4 sind in einer Ebene angeordnet, die parallel zu der ebenen Abtastfläche (in Fig. 1 nicht gezeigt) des Sensors 2 zur Erfassung von Fingerabdrücken verläuft. Aus Gründen der Deutlichkeit sei angenommen, dass die Abtastoberfläche parallel zu der Ebene des Zeichnungsblattes verläuft und dass die Elektroden ebenfalls parallel zu der Abtastoberfläche angeordnet sind.

In dem Ausführungsbeispiel gemäss Fig. 1 sind viele Gruppen von Kondensatoren 4, die in Reihe geschaltet sind, gezeigt. Es sind jedoch lediglich sieben Reihenschaltungen von Kondensatoren im einzelnen gezeigt. Die Reihenschaltungen sind ihrerseits einander parallelgeschaltet. Sie werden aus einer Spannungsquelle 8 versorgt, die eine Spannung V hat. Einer der Kondensatoren, der willkürlich ausgewählt ist, hat die Kapazität $C_i$ und eine Spannung $V_i$. Da alle Kondensatoren das gleiche dielektrische Material aufweisen und da sie alle die gleichen Abmessungen haben, sind ihre Kapazitäten $C_i$ und ihre Spannungen $V_i$ gleich. Dies trifft auf einen Zustand des Sensors zur Erfassung von Fingerabdrücken zu, in dem kein Finger gegen die Abtastoberfläche gepresst wird. Wenn der Finger die Abtastoberfläche berührt, ändert sich die Spannungsverteilung in den Reihenschaltungen der Kondensatoren 4. Die Spannungen $V_i$ der Kondensatoren 4 können mittels einer Multiplexer-Technik abgetastet werden.

Es ist bekannt, dass die Kapazität C eines Kondensators durch die Gleichung

$$C = \frac{kS}{d} \qquad (1)$$

bestimmt ist, wobei C die Kapazität in Farad, k die Dielektrizitätskonstante, S die Fläche des Kondensators in einer Flächeneinheit und d der Abstand zwischen den Elektroden des Kondensators in einer Längeneinheit ist. Es ist ebenfalls bekannt, dass

$$Q = CV \qquad (2)$$

ist, wobei Q die Ladung in Coulomb, C die Kapazität in Farad und V die Spannung in Volt sind.

Aus (1) ist ersichtlich, dass die Kapazität durch eine Veränderung des Abstandes g und/oder der Dielektrizitätskonstanten k verändert werden kann. Die Fläche S des Kondensators ist üblicherweise eine feste Grösse. Aus (2) ist ersichtlich, dass sich die Kondensatorspannung V verändert, wenn die Kapazität C und/oder die Ladung Q verändert wird.

In dem Ausführungsbeispiel gemäss Fig. 1 wird der Abstand d örtlich in Übereinstimmung mit dem Erhöhungs-/-Vertiefungs-Muster des Fingers verändert. In anderen Worten ausgedrückt: Die Kapazitäten der Kondensatoren 4 werden örtlich dann verändert, wenn der abzutastende Finger gegen die Abtastoberfläche gepresst wird. Im Ergebnis wird in der Anordnung gemäss Fig. 1 die Spannungsverteilung in einigen oder allen der Reihenschaltungen von Kondensatoren 4 geändert. Die Änderung der Spannungsverteilungen bzw. der Spannungswerte können durch elektrische Erkennungsmittel, welche im folgenden ins einzelne gehend beschrieben werden, erkannt werden.

Entsprechend Fig. 2 wird ein Finger 12 gegen eine Abtastoberfläche 14 gepresst, die sich in einer Richtung x erstreckt. Der Finger 12 weist Erhebungen 16 und Vertiefungen 18 auf.

Fig. 3 zeigt einen Strang von in Reihe geschalteten Kondensatoren 4. Der Strang wird mit einer Spannung V versorgt. Verglichen mit Fig. 2 sind die Abmessungen der Kondensatoren 4 sehr viel geringer als die Abmessungen der Erhebungen 16 und der Vertiefungen 18 des Fingers 12. Es sei angenommen, dass die Elektrodenplatten der Kondensatoren 4 parallel zu der Abtastoberfläche 14 angeordnet sind.

An den Stellen der Erhebungen nähern sich die Elektroden jedes der Kondensatoren 4 einander. Daraus ergibt sich eine höhere Kapazität gemäss (1). An den Stellen der Vertiefungen 18 der Hautoberfläche werden dagegen die Elektroden nicht so stark zusammengedrückt, so dass sich eine kleinere Kapazität ergibt. Dieser Sachverhalt geht aus Fig. 4 hervor, in der die Verteilung der Kapazitäten $C_i(x)$ in Richtung x gezeigt ist.

In Fig. 5 ist die Spannungsverteilung $V_i(x)$ des Stranges von Kondensatoren 4 gemäss Fig. 3 gezeigt. Durch Vergleich mit Fig. 2 ist ersichtlich, dass die Spannungsverteilung $V_i(x)$ des Stranges von Kondensatoren 4 mit dem Muster von Erhebungen 16 und Vertiefungen 18 der Hautoberfläche des Fingers 12 übereinstimmt.

In anderen Worten: Das Auflegen eines menschlichen Fingers auf die Abtastoberfläche eines kapazitiven Sensors zur Erfassung von Fingerabdrücken bewirkt eine Modulation der Kapazitäts- und Spannungskette.

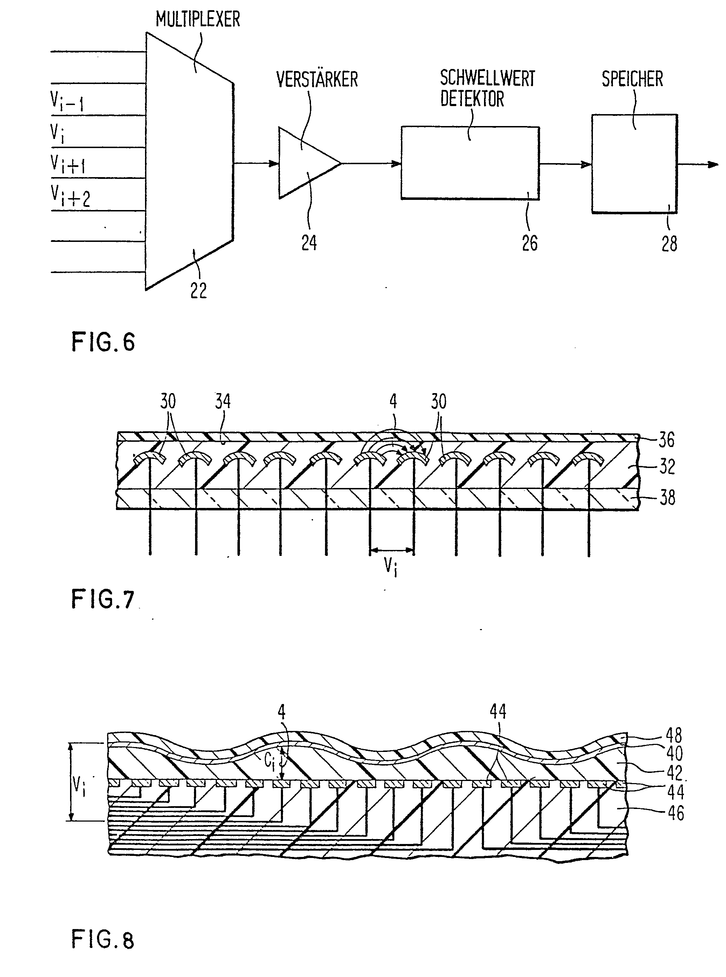

Wie durch eine Lese- und Speicherschaltung in Fig. 6 gezeigt, können die individuellen Spannungen $V_i$, $V_{i+1}$ .... durch Schaltungsmittel einer Multiplextechnik erkannt und verarbeitet werden. Zu diesem Zweck ist ein Multiplexer 22 vorgesehen, dessen Eingänge mit den individuellen Kondensatoren 4 verbunden sind. Der Multiplexer verbindet die Kondensatoren 4 nacheinander mit einem Verstärker 24, der vorzugsweise als Differentialverstärker ausgeführt ist. Der Signalausgang dieses Verstärkers ist mit einem Schwellwertdetektor 26 verbunden. Der Schwellwertdetektor weist einen oder mehrere Schwellwerte auf, mit dem oder denen die jeweils angelegte Spannung verglichen wird. Der Schwellwertdetektor 26 gibt eine binäre Information betreffend die Grösse jedes der Spannungswerte $V_i$, $V_{i+1}$.... ab. Diese binäre Information kann in Speicherzellen eines Speichers 28 abgelegt werden. Diese Information repräsentiert den Fingerabdruck. Der Speicher kann beispielsweise als ladungsgekoppelte Schaltung (CCD-Anordnung) ausgeführt sein. Eine derartige ladungsgekoppelte Schaltung ist auf dem Markt erhältlich. Wenn es gewünscht wird, kann die in dem Speicher 28 enthaltene Information ausgelesen werden. Sie kann durch eine Anzeigeeinrichtung, beispielsweise einen Bildschirm, sichtbar gemacht, ausgedruckt oder mit Hilfe einer Plottervorrichtung dargestellt werden.

Fig. 7 und Fig. 8 zeigen Ausführungsbeispiele für den mechanischen Teil eines Sensors zur Erfassung von Fingerabdrücken gemäss der vorliegenden Erfindung. Sensoren dieser Art sind auf einfache Weise herzustellen, insbesondere dann, wenn Herstellungstechniken, wie sie für die Fertigung von integrierten Schaltkreisen benutzt werden, angewendet werden.

Wie aus Fig. 7 hervorgeht, sind zahlreiche gekrümmte Metallplatten oder Elektroden 30 in einer Abtasteinheit 32 vorgesehen. Diese metallischen Elektroden 30 sind in einer Ebene nahe einer Oberfläche 34 der Abtasteinheit 32 angeordnet. Diese Anordnung ist so getroffen, dass jeweils der äussere Teil der gekrümmten Metallplatte nach der Oberfläche 34 hin ausgerichtet ist. Die Abtasteinheit 32 stellt einen elektrischen Isolator dar.

Die Abtastoberfläche ist von einem Schutzfilm 36 bedeckt, der ebenfalls einen Isolator darstellt.

Jeweils benachbarte gekrümmte Platten bilden einen Kondensator. Einer dieser Kondensatoren ist in der Figur wiederum mit dem Bezugszeichen 4 versehen. Die Spannung $V_i$ jedes dieser Kondensatoren 4 kann gemessen werden, da von ihnen Verbindungsleitungen nach unten durch eine untere Fläche der Abtasteinheit 32 verlaufen. Die Abtasteinheit 32 kann von einer Grundplatte 38 aus einem starren Material getragen werden. Sobald ein Finger die Oberfläche des Schutzfilms 36 berührt, werden die gekrümmten Elektroden 30, die elastisch sind, verformt, wobei die Kapazitäten der Kondensatoren 4 unverändert werden. Die Verteilung der Spannungen $V_i$ der Kondensatoren 4 kann durch die zuvor erwähnte Multiplex-Technik abgenommen und verarbeitet werden.

Es sei darauf hingewiesen, dass in dem Ausführungsbeispiel gemäss Fig. 7 eine Änderung der Dielektrizitätskonstante in Abhängigkeit von der Annäherung des menschlichen Fingers ebenfalls in einer Änderung der Kapazitäts- und Spannungsverteilung resultiert.

Das Ausführungsbeispiel gemäss Fig. 8 nutzt die Gegebenheiten einer festen Dielektrizitätskonstante aus. Dieses Ausführungsbeispiel basiert auf der Änderung des Abstandes d zwischen den Elektroden der individuellen Kondensatoren.

Gemäss Fig. 8 ist eine Abtasteinheit durch eine flexible Elektrode 40, eine flexible Isolationsschicht 42 und eine grosse Anzahl von flachen Metallplatten 44, die von einem Tragteil 46 einer starren Konstruktion gehalten werden, ausgebildet.

Die Metallplatten 44 sind alle in einer Ebene angeordnet, die parallel zu der Oberfläche des Tragteils 46 verläuft. Die flexible Elektrode 40 kann durch eine flexible Membran oder einen Schutzmantel 48 bedeckt sein, der einen elektrischen Isolator darstellt.

Aus Fig. 8 ist ersichtlich, dass die Oberfläche des flexiblen Schutzmantels 48 unter dem Einfluss der Erhebungen und Vertiefungen des (nicht gezeigten) Fingers wellig wird. Die flexible Elektrode 40 folgt dem Muster des Musters von Erhebungen und Vertiefungen des Fingers. Die individuellen Metallplatten 44 bilden Kondensatoren mit der flexiblen Elektrode 40, wobei die flexible Isolationsschicht 42 das dielektrische Material innerhalb dieser Kondensatoren repräsentiert. Einer dieser Kondensatoren ist als Kondensator 4 mit einer Kapazität $C_i$ dargestellt, der eine Spannung $V_i$ aufweist. Jeweils von jeder Metallplatte 44 führen Verbindungsleitungen auf die eine Seite des Sensors zur Erfassung von Fingerabdrücken, wo sie mit einer Leseschaltung (in Fig. 8 nicht gezeigt) verbunden sind. Es ist erkennbar, dass sich der Abstand zwischen der flexiblen Elektrode 40 und den Metallplatten 44 von Kondensator zu Kondensator in Übereinstimmung mit dem Muster, das an der Oberfläche des Sensors entsteht, ändert. Infolge der Änderung des Abstandes verändert sich auch die Kapazität und damit die Spannung jedes der Kondensatoren. Die Spannungsverteilung kann wiederum mittels der Multiplextechnik bemessen und verarbeitet werden.

## Patentansprüche

1. Sensor zur Erfassung von Fingerabdrücken mit einer Abtastoberfläche gegen die der abzutastende Finger gepresst wird, mit Mitteln zum Abtasten der Erhebungen und Vertiefungen in der Hautoberfläche des Fingers, die eine Vielzahl von Kondensatoren (4) enthalten, die in einer Abtasteinheit (32) zusammengefasst und in Form einer zweidimensionalen Matrix angeordnet sind, wobei jeder Kondensator (4) zumindest eine Elektrode (30) aufweist, die mit einem Eingang eines Multiplexers (22) verbunden ist, und mit einer Speichereinrichtung zum Speichern von Ausgangssignalen der Abtasteinheit (32), dadurch gekennzeichnet, dass die Kondensatoren (4) in Form von Reihenschaltungen elektrisch miteinander verbunden sind, dass eine Vielzahl dieser Reihenschaltungen parallelgeschaltet ist, dass die Gesamtheit der Reihenschaltungen aus einer gemeinsamen Spannungsquelle (8) versorgt wird, dass der Multiplexer (22) schaltungstechnisch so angeordnet ist, dass er die Spannungen der Kondensatoren (4) nacheinander abfragt.

2. Sensor nach Anspruch 1, dadurch gekennzeichnet, dass der Ausgang des Multiplexers (22) mit einem Verstärker (24) verbunden ist, der seinerseits an einen Schwellwertdetektor (26) angeschlossen ist, an welchen sich der Speicher (28) anschliesst.

3. Sensor nach Anspruch 1, dadurch gekennzeichnet, dass die Abtasteinheit (32) eine Vielzahl von Metallplatten (30, 44) unter der Abtastoberfläche (34) als Kondensatorelektroden aufweist.

4. Sensor nach Anspruch 3, dadurch gekennzeichnet, dass die Abtasteinheit (32) aus einem elektrischen Isolator besteht, in den die Metallplatten (30) eingebettet sind, und dass die Metallplatten (30) gekrümmt oder V-förmig ausgebildet sind, wobei jeweils benachbarte Metallplatten (30) einen Kondensator (4) bilden.

5. Sensor nach Anspruch 3, dadurch gekennzeichnet, dass die Abtasteinheit eine flexible Elektrode (40) auf einer flexiblen Isolationsschicht (42) aufweist, die Metallplatten (44) in einer Ebene auf einem starren Tragteil angeordnet sind, die flexible Isolationsschicht (42) zwischen der Ebene der Metallplatten (44) und der flexiblen Elektrode (40) angeordnet ist und die Metallplatten mit der flexiblen Elektrode die einzelnen Kondensatoren (4) bilden.

6. Sensor nach einem der Ansprüche 3 bis 5, dadurch gekennzeichnet, dass die Abtastoberfläche (14, 34) mit einem Schutzfilm (36, 48) bedeckt ist.

7. Sensor nach einem der Ansprüche 1 bis 6, dadurch gekennzeichnet, dass die Abmessungen der Kondensatoren (4) zumindest um den Faktor 5 kleiner als die durchschnittliche Breite der Erhebungen (16) und Vertiefungen (18) des Fingers (12) sind.

## Claims

1. A sensor for detecting finger-prints, with a scanning surface against which the finger to be scanned is pressed, with means which serve to scan the elevations and recesses in the main surface of the finger and contain a plurality of capacitors (4) assembled in a scanning unit (32) arranged in the form of a two-dimensional matrix, where each capacitor (4) is provided with at least one electrode (30) connected to an input of a multiplexer (22), and with a storage device which serves to store output signals from the scanning unit (32) characterised in that the capacitors (4) are electrically connected to one another in the form of series circuits, that a plurality of these series circuits are connected in parallel, that all the series circuits are supplied from a common voltage source (8), and that the multiplexer (22) is arranged in a circuit such that it consecutively interrogates the voltages of the capacitors (4).

2. A sensor as claimed in Claim 1, characterised in that the output of the multiplexer (22) is connected via an amplifier (24) to a threshold value detector (26) which is in turn connected to the store (28).

3. A sensor as claimed in Claim 1, characterised in that the scanning unit (32) contains a plurality of metal plates (30, 44) arranged under the scanning surface (34) as capacitor electrodes.

4. A sensor as claimed in Claim 1, characterised in that the scanning unit (32) consists of an electric insulator in which the metal plates (30) are embedded, and that the metal plates (30) are of curved or V-shaped formation, and adjacent metal plates (30) in each case form a capacitor (4).

5. A sensor as claimed in Claim 3, characterised in that the scanning unit contains a flexible electrode (40) on a flexible insulation layer (42), the metal plates (4) are arranged in one plane on a rigid bearing component, the flexible insulation layer (42) is arranged between the plane of the metal plates (44) and the flexible electrode (40), and the metal plates together with the flexible electrode form the individual capacitors (4).

6. A sensor as claimed in one of Claims 3 to 5, characterised in that the scanning surface (14, 34) is covered by a protective film (36, 48).

7. A sensor as claimed in one of Claims 1 to 6, characterised in that the dimensions of the capacitors (4) are at least five times smaller than the average width of the elevations (16) and recesses (18) of the finger (12).

## Revendications

1. Capteur servant à détecter des empreintes digitales, comportant une surface d'exploration contre laquelle le doigt devant être exploré est appuyé, et comportant des moyens pour explorer les élévations et les renfoncements situés à la surface de la peau du doigt, qui contiennent un grand nombre de condensateurs (4) qui sont réunis dans une untité d'exploration (32) et sont disposés sous la forme d'une matrice bidimensionnelle et dont chacun comporte au moins une électrode (30) qui est reliée à une entrée d'un multiplexeur (22), et comportant un dispositif de mémoire servant à mémoriser des signaux de sortie de l'unité d'exploration (32), caractérisé par le fait que les condensateurs (4) sont reliés électriquement entre eux sous la forme de circuits série, qu'un grand nombre de ces circuits série sont branchés en parallèle, que la totalité des circuits série est alimentée par une source commune de tension (8) et que le multiplexeur (22) est disposé du point de vue de la technique des circuits de telle sorte qu'il interroge successivement les tensions des condensateurs (4).

2. Capteur suivant la revendication 1, caractérisé par le fait que la sortie du multiplexeur (22) est reliée à un amplificateur (24) qui pour sa part est raccordé à un détecteur à valeur de seuil (26), auquel est raccordée la mémoire (28).

3. Capteur suivant la revendication 1, caractérisé par le fait que l'unité d'exploration (32) comporte un grand nombre de plaques métalliques (30, 44) situées au-dessous de la surface d'exploration (34) et constituant des électrodes des condensateurs.

4. Capteur suivant la revendication 3, caractérisé par le fait que l'unité d'exploration (32) est constituée par un isolateur électrique, dans lequel sont logées les plaques métalliques (30), et que les plaques métalliques (30) sont réalisées avec une forme cintrée ou une forme de V, des plaques métalliques (30) respectivement voisines constituant un condensateur (4).

5. Capteur suivant la revendication 3, caractérisé par le fait que l'unité d'exploration comporte une électrode flexible (40) située sur une couche isolante flexible (42), que les plaques métalliques (44) sont disposées dans un plan sur un organe de support rigide, que la couche isolante flexible (42) est disposée entre le plan des plaques métalliques (44) et l'électrode flexible (40) et que les plaques métalliques forment avec l'électrode flexible les différents condensateurs (4).

6. Capteur suivant l'une des revendications 3 à 5, caractérisé par le fait que la surface d'exploration (14, 34) est recouverte par une pellicule protectrice (36, 48).

7. Capteur suivant l'une des revendications 1 à 6, caractérisé par le fait que les dimensions des condensateurs (4) sont inférieures, au moins du facteur 5, à la largeur moyenne des élévations (16) et des renforcements (18) du doigt (12).

FIG.1

$C_i$

$C_i+1$

$V_i$

8

+V

FIG. 2

FINGER

12

18    $X_i$    16    18    16    18    14    X

o +V

4

$V_i$

FIG. 3    $X_i$    X

$C_i$

FIG. 4    $X_i$    X

$V_i$

FIG. 5    $X_i$    X

7

MULTIPLEXER

VERSTÄRKER

SCHWELLWERT
DETEKTOR

SPEICHER

$V_{i-1}$

$V_i$

$V_{i+1}$

$V_{i+2}$

24

26

28

22

## FIG.6

30   34

4   30

36

32

38

$V_i$

## FIG.7

4

44

48
40
42
44
46

$c_i$

$V_i$

## FIG.8